# EUROPEAN PATENT APPLICATION

(11) **EP 4 238 413 A1**
(43) Date of publication of application: **06.09.2023**
(21) Application number: 22159929.3
(22) Date of filing: 03.03.2022
(51) Int. Cl.: A01K 67/033

(54) **REBATCHING INSECT EGGS INTO A BREED CRATE**

(71) Applicant: Bühler AG, 9240 Uzwil (CH)
(72) Inventor: Wooding, Vaughan, 9230 Flawil (CH)
(74) Representative: Vossius & Partner Patentanwälte Rechtsanwälte mbB

(57) **Abstract**

The present invention relates to a method and a system for rearing insects, with the steps of breeding insects in a breed crate, such that the breed crate comprises live insects, substrate and insect eggs, emptying the breed crate for sorting its content by separating the live insects and collecting a mixture of the substrate mixed with said insect eggs, and rebatching the collected egg/substrate mixture to a breed crate for further hatching the eggs in the mixture and rearing the emerging neonates.

## Description

The present disclosure relates to a method for rearing insects and a corresponding system. In particular, the disclosure relates to rebatching insect eggs into a breed crate.

When breeding insects, e.g. mealworms, the adult beetles lay eggs into the substrate (feed) and against the side walls of the crate or container, before the adult insects are removed in order to continue the breeding cycle with the eggs. From an efficiency and economical point of view, it is crucial to minimize egg losses during this process. According to the state of the art, breeders use an insert inside the breed crate to reduce egg loss during breeding and removal of adult insects. The eggs are collected in a space between the crate bottom and the insert is removed together with the adult insects and substrate. However, this still results in significant egg losses, since some of the eggs are placed on the insert or the side walls of the insert and are therefore removed together with the insert which is subsequently cleaned. Moreover, the insert adds many technical challenges and costs to an insect rearing system.

The present disclosure has been made in view of the above problems. In particular, the present disclosure provides a method and system for rearing insects without an insert while minimizing egg losses.

The invention is defined in the independent claims. Dependent claims describe preferred embodiments.

The present invention relates to a method for rearing insects, with the steps of
(a) breeding insects in a breed crate, such that the breed crate comprises live insects, substrate and insect eggs,
(b) emptying the breed crate for sorting its content by separating the live insects and collecting a mixture of the substrate mixed with said insect eggs, and
(c) rebatching the collected mixture to a breed crate for further hatching the eggs in the mixture and rearing the emerging neonates.

Various embodiments may preferably implement the following features:
The substrate may include frass, feed, insect skins or a mixture of feed, frass and skins.

During step (b) or as an additional step, the eggs may further be separated from the mixture of eggs and substrate, and the eggs may subsequently be mixed back to the substrate in a different, preferably higher amount, leading to an improved egg/substrate mixture that may be used for rebatching.

Preferably, in step (b) the breed crate is emptied by tipping the breed crate and/or using a suction means. By using tipping, the crates are emptied by gravity, which is a simple and efficient way to empty a crate. Suction has the advantage that a crate can be emptied independent of gravity and gravity forces.

Preferably, in step (b) sorting the content comprises sieving and/or air separation of the content of the breed crate. Live insects have a different size and density compared to substrate and eggs. That is why they can be efficiently separated using sieving, which separates according to size, and/or air separation, which separates according to density. That way, the separation of live insects can be managed without the need of an insert in the breed crate, which gives an advantage for large-scale, automated facilities, because no equipment for handling the insert is needed.

Preferably, the method further comprises removing dead insects during and/or after step (b). Dead insects do not contribute to the nutrition of insects, but still occupy a certain space. Thus, removal of dead insects from substrate and egg mixture reduces the volumetric space requirements when the egg/substrate mixture is rebatched to a breed crate for further hatching the eggs and rearing the emerging neonates. Presence of dead insects can also reduce reproduction potential of adult insects or provide a breeding ground for harmful pathogens.

Preferably, in step (c) the collected egg/substrate mixture is refilled into the breed crate used for breeding in step (a). Some eggs might be still attached to the walls of the breed crate. By using the same breed crate, these eggs are brought back to the process. This reduces the overall egg losses to nearly zero.

Preferably, the egg/substrate mixture of a plurality of breed crates is collected in step (b) and re-dosed back into the breed crates by weight or volume in step (c). This allows to change the egg/substrate amount to be added to a breed crate. In addition, the same amounts of egg/substrate mixture can be assured in each breed crate. Finally, dosing by weight and/or volume allows for quick and sufficiently accurate dosing without resorting to difficult or tedious techniques such as egg or neonate counting.

Preferably, the breed crates from step (a) and reused in step (c) are not washed before refilling. Washing of the crate removes and likely kills the eggs that were stuck to the side walls and floors of the crate resulting in loss of egg yield which in turn increases the required adult insects. Preferably, in step (c) fresh substrate is added to the egg/substrate mixture collected in step (b). The ability to add fresh substrate to the egg/substrate mixture allows to provide additional nutrition for the rearing of the emerging neonates after egg hatching. The fresh substrate can improve the adaptation of the young animals to the substrate and the initiation of a quick growth.

The method preferably further comprises collecting the separated live insects for further processing. Some adult insects might still be of value in the breed cycle and can be reused, while other adult insects might not enter back to the breed cycle. This means that the processing of the separated live insects collected in step (b) might comprise a processing step to recover adult insects to be reused in the breed cycle. In addition, adult insects can be turned into sellable products and processing in this case might contain steps as grinding, drying, and/or chitin extraction among others.

The collected live insects are preferably transferred to breed crates filled with fresh substrate for further rearing or mating of the insects. Retention of adults for reuse in breed cycle reduces the total number of larval stage insects that need to be reared to adulthood to continue the colony. To exploit this potential, collected adult insects for reuse are transferred to breed crates filled with fresh substrate to continue egg laying. This increases the overall efficiency of the facility.

The present disclosure also encompasses a corresponding system for rearing insects, preferably using the method as described above. The system comprises a breed crate configured to contain live insects, substrate and insect eggs, a sorting means configured to empty the breed crate for sorting its content by separating the live insects and collecting a mixture of the substrate mixed with said insect eggs and a rebatching means for transferring the collected mixture to a breed crate.

Preferably, the sorting means comprises a crate tipper or a suction means for emptying the breed crate. Both systems - crate tipper and suction means - allow an efficient and cost-effective emptying of the breed crate.

Preferably, the sorting means comprises a sieve or an air separation means for separating the live insects from the mixture of the substrate and the insect eggs. Sieving and air separation provide efficient and cost-effective separation of live insects from the egg/substrate mixture. The two techniques are also suitable to be applied on large scale.

The system preferably further comprises collecting means for collecting the egg/substrate mixture of a plurality of breed crates and dosing means for dosing the collected mixture into the crates by weight or volume. This allows for product buffering and also increases process flexibility in the case where a 1 to 1 crate transfer is not feasible.

The rebatching means is preferably configured to refill the egg/substrate mixture into previously emptied breed crates. This allows for breed crates to move into the next stage of the rearing cycle without being washed in order to optimise egg yield.

The disclosure will be further described with reference to the attached drawings.

Figs. 1 and 2 show exemplary flow charts according to the present disclosure;

Fig. 3 schematically shows an air aspirator used for the present invention.

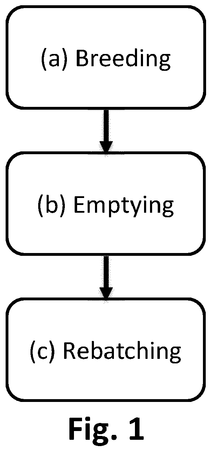

Fig. 1 shows a basic flow chart of the method according to the present disclosure. The method comprises step (a) breeding insects in a breed crate, such that the breed crate comprises live insects, substrate and insect eggs. The substrate may include feed, frass, insect skins or a mixture of feed, frass and insect skins. In step (b), the breed crate is emptied for sorting its content by separating the live insects and collecting a mixture of the substrate mixed with said insect eggs. Subsequently, in step (c), the collected mixture is rebatched to a breed crate for further hatching the eggs in the mixture and rearing the emerging neonates.

The term rebatching thus refers to refilling the collected egg/substrate mixture into the breed crate without previously cleaning the breed crate. The method is not restricted to a particular kind of insects.

Step (b) of emptying the breed crates may be performed by tipping the crate. Alternatively or in addition, a vacuum means or suction means may be utilised for emptying the breed crate. Since the breed crates are rebatched, they do not need to be emptied completely. According to the proposed method and system, eggs and substrate remaining in the breed crate after the emptying step are then used for the hatching and rearing of insects.

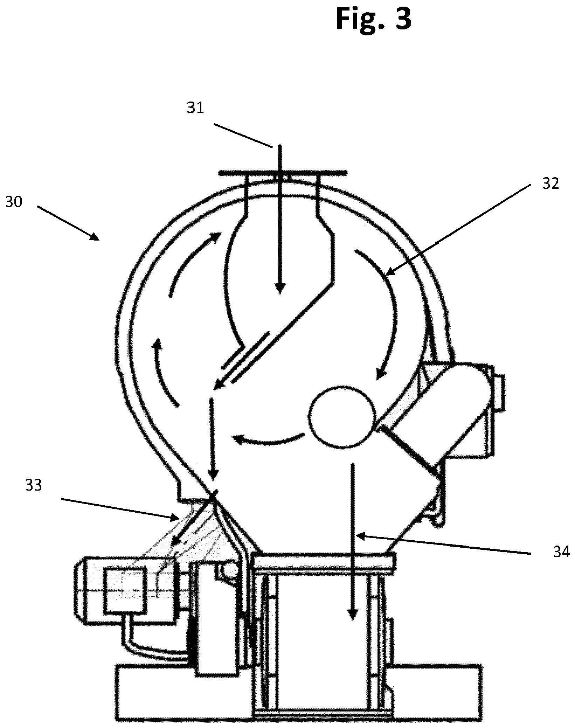

In order to separate the live insects from their eggs (and the substrate), the content of the breed crate extracted in the emptying step (b) may be sieved. In addition or alternatively, the content of the breed crate may be provided to an air separation means, such as schematically shown in Fig. 3. The air separation means 30 performs an air sieving depending on the density of the provided particles. Since the density of adult or hatched insects differs from the density of eggs and/or substrate, this may be an effective and gentle way of sorting. By using an air aspirator, such as an air-recycling aspirator of Bühler AG, originally designed for removing fine particles from grains, clogging can be prevented also in this use, whereby a more effective separation takes place. Effective air separation removes the fine particles without harming or damaging the live insects. The air aspirator 30 can effectively remove fine particles and grade the insects by life-stage, a sieving step can further grade the insects by size. It can therefore act as an automated system with no operator or human interaction, and insects and eggs can be effectively separated. The ability to create multiple stages of separation further reduces losses. The air separation can be done by blowing or suction.

For separation, the content of the breed crate may be inputted into the air siever 30 through an input 31. By use of a circulating air flow inside the air separating means 30, indicated by arrow 32, the mixture can be separated into hight density particles, i.e. the live insects, that are substantially not influenced by the air flow 32 and can be extracted though port 33, and low density particles, i.e. frass and eggs, that are guided by the air flow to exit 34

During step (b), dead insects may be removed from the content. Said removal may also be performed after step (b). In step (c), the collected egg/substrate mixture may be refilled into the breed crate used for breeding in step (a). According to an embodiment of the invention the eggs may further be separated from the egg/substrate mixture, and may subsequentially be mixed with a different, and preferably lower amount of substrate. In particular, this may lead to an improved egg/substrate mixture with a higher proportion of eggs for subsequent rebatching.

The egg/substrate mixture of a plurality of breed crates may be collected in step (b) and re-dosed back into the breed crates by weight or volume in step (c). The breed crates from step (a) and reused in step (c) may not be washed before refilling. In step (c), fresh substrate, in particular feed, may be added to the mixture.

The rebatching, i.e. refilling of the egg and substrate mixture in a breed crate, may therefore be performed in the same breed crate that has just been emptied or another empty crate. Moreover, the content of a plurality of crates may be collected and then dosed into a crate for hatching eggs and rearing the emerging neonates. This may depend on facility properties and the process flow (e.g. continuous/batch processing).

The separated live insects may be collected for further processing. In particular, the collected live insects may be transferred to breed crates filled with fresh substrate for further rearing or mating of the insects.

Accordingly, the present disclosure encompasses a corresponding system for rearing insects comprising a breed crate 1 configured to contain live insects, substrate and insect eggs, a sorting means 2, 3 configured to empty the breed crate for sorting its content by separating the live insects and collecting a mixture of the substrate mixed with said insect eggs, and a rebatching means 4 for transferring the collected mixture to a breed crate.

The sorting means may comprise a crate tipper 2 for emptying the breed crate. In addition or alternatively, the crates may be emptied using a suction means 2 or vacuum means, i.e. a suction device for emptying the crates 1. The sorting means may comprise a sieve 3 and/or an air separation means 3 as described above for separating the live insects from the mixture of the substrate and the insect eggs.

The system may further comprise collecting means for collecting the egg/substrate mixture of a plurality of breed crates 1 and dosing means for dosing the collected mixture into the crates 1 by weight or volume. The rebatching means 4 may be configured to refill the egg/substrate mixture into previously emptied breed crates. The collecting means and the dosing means may also form part of the rebatching means 4. The collecting means may e.g. be a crate 1, a container or a trough and the dosing means may e.g. be a weighing station, a volume measuring device or a flow rate measuring device. The collecting means may also be placed on a weighing device in order to control the content of the crates 1.

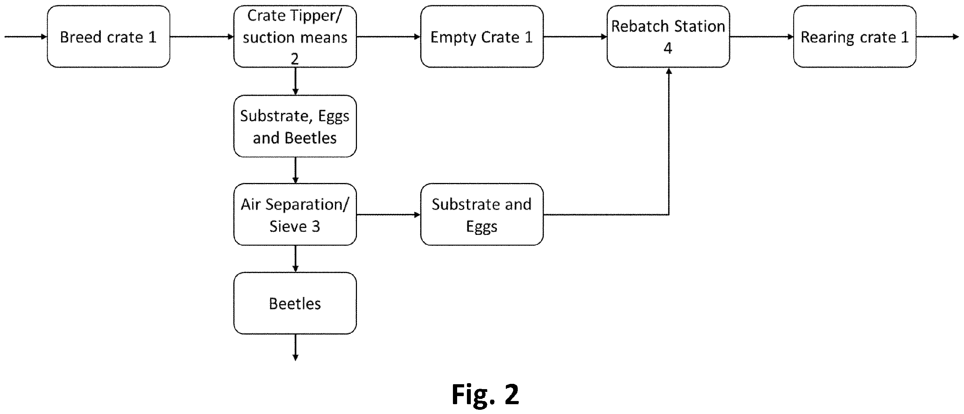

Fig. 2 shows an exemplary overview and flow chart of the present disclosure. Fig. 2 is fully compatible with the above description and uses beetles as live insects as an example only.

According to Fig. 2, a breed crate 1 containing live insects, substrate and insect eggs (step (a)) is provided to a crate tipper 2 and/or a suction means 2. Thus, an empty crate 1, which still may contain substrate, eggs and/or insect residues, and a composition of substrate, eggs and beetles is obtained. As explained above, the term substrate may refer to frass, feed, insect skins or a mixture of frass, feed and skins. The empty crate 1 is not cleaned in order not to remove eggs which are still present after the tipping or suction process. The composition is then provided to an air separation device 3 or a sieve 3, where live insects and a mixture of substrate and eggs are collected (step (b)). The separated live insects, in this case beetles, are further processed or provided to another crate for laying eggs. The collected mixture of substrate and eggsis then provided to a rebatch station 4 as explained above. In the rebatch station 4, the mixture of substrate and eggs is provided to a breed crate 1 for further hatching the eggs in the mixture and rearing the emerging neonates (step (c)). Thus, the breeding crate 1 becomes a rearing crate 1 and loss of eggs or insects can be avoided.

According to the present disclosure, a method and a system is proposed which reduces the amount of egg loss during a rearing process while ensuring food safety and gentle handling of the insects. Therefore, the breed crate becomes the rearing crate even though the contents of the breed crate are tipped out. The tipping contents are sieved or processed by air separation to remove the beetles and then rebatched into the crate.

Hence, an efficient, high-output rearing method and system are provided which minimise egg losses by rebatching a sorted mixture of eggs and substrate into a breed crate without the necessity of previously cleaning the crate.

Other aspects, features, and advantages will be apparent from the summary above, as well as from the description that follows, including the figures and the claims.

While the invention has been illustrated and described in detail in the drawings and foregoing description, such illustration and description are to be considered illustrative or exemplary and not restrictive. It will be understood that changes and modifications may be made by those of ordinary skill within the scope of the following claims. In particular, the present invention covers further embodiments with any combination of features from different embodiments described above and below.

Furthermore, in the claims the word "comprising" does not exclude other elements or steps, and the indefinite article "a" or "an" does not exclude a plurality. A single unit may fulfil the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

## Claims

1. Method for rearing insects, with the steps of
(a) breeding insects in a breed crate, such that the breed crate comprises live insects, substrate and insect eggs,
(b) emptying the breed crate for sorting its content by separating the live insects and collecting a mixture of the substrate mixed with said insect eggs, and
(c) rebatching the collected mixture to a breed crate for further hatching the eggs in the mixture and rearing the emerging neonates.

2. Method according to claim 1, wherein the breed crate is emptied by tipping the breed crate and/or using a suction means.

3. Method according to claim 1 or 2, wherein sorting the content comprises sieving and/or air separation of the content of the breed crate.

4. Method according to any one of the preceding claims, further comprising removing dead insects during and/or after step (b).

5. Method according to any one of the preceding claims, wherein in step (c) the collected egg/substrate mixture is refilled into the breed crate used for breeding in step (a).

6. Method according to any one of claims 1 to 4, wherein the egg/substrate mixture of a plurality of breed crates is collected in step (b) and re-dosed back into the breed crates by weight or volume in step (c).

7. Method according to any one of the preceding claims, wherein the breed crates from step (a) and reused in step (c) are not washed before refilling.

8. Method according to any one of the preceding claims, wherein in step (c) fresh substrate is added to the collected egg/substrate mixture from step (b).

9. Method according to any one of the preceding claims, further comprising collecting the separated live insects for further processing.

10. Method according to claim 9, wherein the collected live insects are transferred to breed crates filled with fresh substrate for further rearing or mating of the insects.

11. System for rearing insects, preferably using the method of any of the preceding claims, the system comprising
a breed crate configured to contain live insects, substrate and insect eggs,
a sorting means configured to empty the breed crate for sorting its content by separating the live insects and collecting a mixture of the substrate mixed with said insect eggs, and
a rebatching means for transferring the collected egg/substrate mixture to a breed crate.

12. System according to claim 11, wherein the sorting means comprises a crate tipper and/or a suction means for emptying the breed crate.

13. System according to claim 11 or 12, wherein the sorting means comprises a sieve or an air separation means for separating the live insects from the mixture of the substrate and the insect eggs.

14. System according to claim 11 to 13, further comprising collecting means for collecting the egg/substrate mixture of a plurality of breed crates and dosing means for dosing the collected mixture into the crates by weight or volume.

15. System according to any one of claims 11 to 14, wherein the rebatching means is configured to refill the collected egg/substrate mixture into previously emptied breed crates.
